# EUROPEAN PATENT SPECIFICATION

(11) **EP 1 838 805 B1**
(45) Date of publication and mention of the grant of the patent: **02.12.2009**
(21) Application number: 05701477.1
(22) Date of filing: 10.01.2005
(51) Int. Cl.: C09K 11/06, C07D 471/00

(54) **ORTHOGONALLY FIXED COMPOUNDS FOR ELECTROOPTICAL APPLICATIONS**
ORTHOGONAL FIXIERTE VERBINDUNGEN FÜR ELEKTROOPTISCHE ANWENDUNGEN
COMPOSES A LIAISON ORTHOGONALE POUR APPLICATIONS ELECTRO-OPTIQUES

(43) Date of publication of application: 03.10.2007
(73) Proprietor: Technische Universität Braunschweig, 38106 Braunschweig (DE)
(72) Inventor: JOHANNES, Hans-Hermann, 38124 Braunschweig (DE); KOWALSKY, Wolfgang, 38116 Braunschweig (DE); DÜMELAND, Martin, 06114 Halle (DE); LAWRENTZ, Ulf, 90765 Fürth (DE); KRÖNER, Michael, 67551 Worms-Pfeddersheim (DE)
(74) Representative: Taruttis, Stefan Georg
(86) International application number: PCT/EP2005/050086
(87) International publication number: WO 2006/072470

(56) References cited:
- US-A- 5 840 217
- US-A1- 2005 003 286

## Description

The present invention relates to chemical compounds that can be used in electrochemical or electrooptical applications. The electric and/or optical properties of these compounds, e.g. hole transporting, electron transporting, hole injecting, electron injecting and/or light emitting properties can be predetermined by substituting the core structure of these compounds with respective residues. Electric and electrooptic applications comprise organic light emitting diodes (OLEDs), organic field effect transistors (OFETs), lasers, and photovoltaic devices suitable for photovoltaic solar energy conversion.

The core structure of the compounds according to the invention comprises two opposing aromatic moieties which are chemically bonded through an intermediate central atom having a tetraedric configuration to provide an orthogonal orientation to the bonded aromatic moieties.

### State of the art

WO 96/17035 discloses heterospiro compounds and their use as electroluminescent materials, generally formed of two conjugated systems which are directly linked by a central atom, for example silicon, germanium or tin. Furthermore, there is disclosed a heterospiro compound having two biphenyl groups as symmetrical aromatic moieties which are linked via the central tetraedric atom to form a spiro compound. The biphenyl groups are linked to one another by the intermediate central atom, each biphenyl group forming a fluorene structure with its two phenyl moieties and the central atom.

EP 0676461 A2 discloses compounds analogous to WO 96/17035, wherein the central atom is a carbon atom.

### Objects of the invention

It is an object of the invention to provide compounds which are an alternative to known spiro compounds. It is preferred that the alternative compounds have a high glass transition temperature and good long term stability. Further, it is preferred that the alternative compounds can be derivatized to introduce the electrical and/or luminescent properties desired for electrooptical applications.

### General description of the invention

The present invention achieves the above-mentioned object by providing the core structure of general formula I for compounds suitable for electrooptical and/or electroluminescent applications: 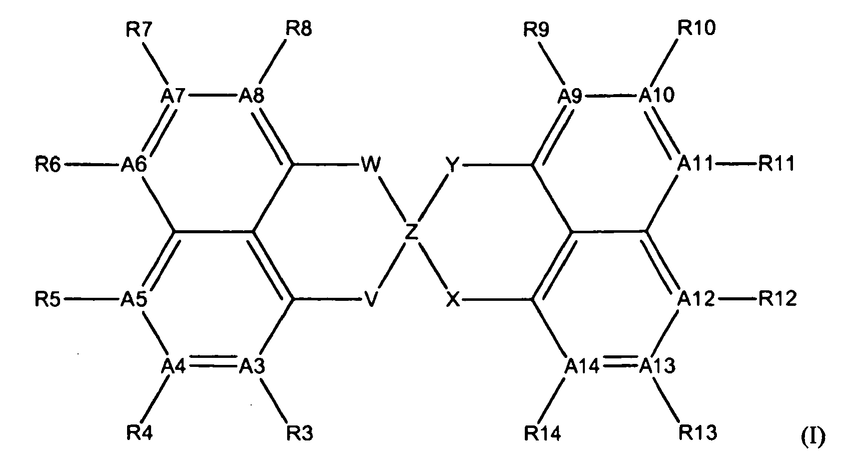
wherein V, W, X and Y can be selected from at least divalent atoms or groups, e.g. -S-, -NR-, -O-,
a carbonyl group, -SO₂-, and di-substituted silicon, -CRR-, and a chemical bond, with R any (hetero-) alkyl or (hetero-) aryl or hydrogen, wherein at least one of V, W and X, Y, respectively are no chemical bonds but atoms,
wherein R3 to R14 are independently selected from (hetero-) alkyls, (hetero-) aryls, -NR'₂, -OR', -SR', -CN, -F, -CF₃, with R' independently selected from substituted or unsubsituted (hetero-)alkyl, (hetero-)aryl or hydrogen, and electrooptically functional groups, wherein two or more of R3 to R14 can be condensed arenyl groups or groups forming a higher condensed derivative of the core structure of formula I,
wherein A3 to A14 each are a carbon atom, and
wherein Z is a central tetraedric atom.

The central atom Z may be formed by silicon, germanium and, preferably, carbon.

Core structure I comprises a first and a second condensed aromatic system which are connected via central atom Z. Preferably, the first and second condensed aromatic systems are arranged at central atom Z in a position essentially opposite one another. The condensed aromatic systems are each linked to the central atom Z through their adjacent a, α' carbon atoms and by intermediate residues V, W, arranged between the α carbon atoms of the first condensed aromatic system and central atom Z and intermediate residues X, Y, respectively, arranged between the α' carbon atoms of the second condensed aromatic system and central atom Z.

Accordingly, the linkage of the first and second condensed aromatic systems to central atom Z is independently formed as a four-, five- or six-membered ring including the α carbon atoms or the α' carbon atoms, which are part of the first condensed aromatic system and of the second condensed aromatic system, respectively.

In one embodiment, one of intermediate residues V, W linking the a carbon atoms of the first condensed aromatic system to central atom Z is an atom, whereas the other intermediate residue W, V, respectively, is a chemical bond, directly linking one of both α carbon atoms to the central atom Z, forming a five-membered ring which comprises central atom Z, one intermediate residue and the α carbon atoms of the first condensed aromatic system. In a first alternative embodiment, both intermediate residues V, W are atoms, same or different, each arranged between one of both α carbon atoms of the first condensed aromatic system and central atom Z, forming a six-membered ring comprising the α carbon atoms of the first condensed aromatic system, both intermediate residues and central atom Z. In a second alternative embodiment, both V and W are single bonds, forming a four-membered ring, directly linking central atom Z to both α carbon atoms.

Independent from the embodiment of the linkage of the first condensed aromatic system to the central atom, the second condensed aromatic system is linked to the central atom with at least one intermediate residue X, Y being an at least divalent atom or residual group. In one embodiment, the second condensed aromatic system is linked to central atom Z through its α' carbon atoms with one of intermediate residues X, Y being an atom and the other one of Y, X, respectively, being a chemical bond, forming a five-membered ring between the condensed aromatic system and central atom Z including either intermediate residue X or Y. In an alternative embodiment, both intermediate residues X, Y, respectively are atoms, each arranged between one of the α' carbon atoms of the second condensed aromatic system and the central atom Z, forming a six-membered ring.

In a preferred embodiment, one of or both of intermediate residues V, W, and x Y, respectively, are di-substituted carbon atoms, preferably methylene groups. Alternatively, one of V, W and X, Y, respectively, is a di-substituted carbon atom preferably CR1R2, whereas the other intermediate residue is sulfur, oxygen or a non-substituted or mono-substituted nitrogen.

The bonds between each of the condensed aromatic systems and the central atom are nonconjugated bonds, providing for electronic isolation of the first and second condensed aromatic systems. The respective substituents can be linked conjugatedly or non-conjugatedly to their respective condensed aromatic systems.

The condensed aromatic systems of the core structure may form part of higher anellated aromatic moieties, for example a naphthyl moiety that provides α and α'carbon atoms for linkage to central atom Z may be comprised in an anthracene moiety, a naphthacene or a pentacene moiety as well as in a phenanthrene, chrysene, acenaphthylene, pyrene, coronene, benzo(a)pyrene, or naphthopyrene moiety, providing carbon atoms in positions α and α'.

The central structure according to general formula I provides the compounds according to the invention with the advantageous properties of having a low propensity to crystallize, which is reflected in a high glass transition temperature. High glass transition temperatures are desired for compounds according to the invention in electrical, especially in electrooptical applications. It is assumed that the steric confirmation of the central structure, arranging the first and second condensed aromatic systems in an orthogonally orientated position is the cause for the advantageous properties of compounds according to the invention.

Substituent groups R3 through R14 can be electrooptically functional groups like hole injecting moieties or hole transporting moieties, electron injecting moieties or electron transporting moieties, and/or luminescence emitting moieties, or non-EL groups like (hetero-) alkyl and (hetero-) aryl groups unless they represent higher aromatic substituents which in combination with the respective condensed aromatic system form higher anellated systems like anthracene, naphthacene, pentacene, phenanthrene, chrysene, acenaphthylene, pyrene, coronene, benzo(a)pyrene, naphthopyrene or condensates thereof. However, at least one substituent having EL properties is linked to a condensed aromatic system, the EL functions provided in each condensed aromatic system may be selected independently.

EL functional group residues conferring hole transporting characteristics onto the core structure of general formula I can be selected from tris-[(*N,N*-diaryl)amino]-triphenylamines like 4,4',4"-tris[(*N*-(1-naphthyl)-*N*-phenyl-amino-triphenylamine] (1-TNATA) and its derivatives, 4,4',4"-tris[(*N*-(2-naphthyl)-*N*-phenyl amino)-triphenylamine] (2-TNATA) or 4,4',4"-tris[(*N*-(3-methylphenyl)-*N*-phenyl-amino)-triphenylamine] (m-TDATA) and its derivatives, 4,4',4"-tris(carbazole-9-yl)triphenylamines; *N,N*,*N',N'*-tetraarylbenzidines as *N,N*,*N',N'*-tetraphenylbenzidine and its derivatives, *N,N*'-bis(1-naphthyl)-*N,N*'-diphenylbenzidine (α-NPD), *N,N*'-di(naphthalene-2-yl)-*N,N*'-diphenylbenzidine (β-NPD), 4,4'-bis(carbazole-9-yl)biphenyl (CBP) and its derivatives, and their heteroatom substituted analogs (e.g. thienyl-, selenyl-, furanyl-derivatives); 4,4'-bis(2,2'-diphenylvinyl)-1,1'-biphenyl (DPVBI); triarylamines and their derivatives, 4,4'-bis(*N,N*-diarylamino)-terphenyls, 4,4'-bis(*N,N* diarylamino)-quarterphenyls and their homologs and derivatives.

EL functional group residues conferring electron transporting characteristics onto the core structure of general formula I can be selected from 4,7-diphenyl-1,10-phenanthroline (Bphen) and derivatives thereof as 2,9-dimethyl-4,7-diphenyl-1,10-phenanthroline (BCP), 2,5-diaryloxadiazoles and derivatives thereof as 2-(*p*-*tert.*butylphenyl)-5-(*p-*biphenyl)-oxadiazole (PBD), oligo-(benzoxadiazol-2yl)-arenes and derivatives thereof as bis-2,5-(5-*tert.*-butylbenzoxadiazol-2-yl)-thiophene (BBOT), 1,3-bis[5-(aryl)-1,3,4-oxadiazol-2yl]benzenes and derivatives thereof as 1,3-bis[5-(*p-tert.*-butylphenyl)-1,3,4-oxadiazol-2yl]benzene (OXD-7), 2,5-diaryltriazoles and derivatives like 2-(*p-tert-*butylphenyl)-5-(*p-*biphenyl)-triazole (TAZ).

EL functional group residues conferring emitter characteristics onto the core structure of general formula I can be selected from residues which in combination with the condensed aromatic system result in a dye. Dyes resulting from this combination may for example be coumarines, rhodamines, merocyanines, e.g. derivatives of DCM, DCM2, cyanines or oxonoles.

In the alternative to the one or more EL functional group residue(s) being directly linked to the condensed aromatic system, it can be linked to the condensed aromatic system via intermediate residue groups, for example condensed rings, or other linker groups, e.g. (betero-) alkyl or (hetero-) aryl groups.

Using compounds according to the invention, various EL devices can be constructed. The inventive compounds have the advantage that the core structure according to general formula I can be adapted by pre-selecting its substituents for specific EL functions, generating a compound with specific EL properties. From these compounds, layers in EL devices can be deposited from solution or by vapour deposition having pre-determined electrical and/or optical properties. Compounds comprising this core structure share the advantage of having high glass transition temperatures, a good solubility in organic solvents and, preferably, also a high long term stability.

### Detailed description of the invention

The invention will now be described with reference to the figures, wherein
- Figure 1 describes a compound according to the invention having hole transporting properties,
- Figure 2 shows the structure of a compound according to the invention having hole transporting properties,
- Figure 3 shows a compound according to the invention having electron transporting properties,
- Figure 4 shows a compound according to the invention having electron transporting properties,
- Figure 5 shows a compound according to the invention having hole transporting properties,
- Figure 6 shows a compound according to the invention having hole transporting properties,
- Figure 7 shows a compound according to the invention having the property of electron transport,
- Figure 8 shows a compound according to the invention having combined properties of hole transport and electron transport,
- Figure 9 shows a compound according to the invention having emitter properties,
- Figure 10 shows a compound according to the invention having combined properties for hole and/or electron transport,
- Figure 11 shows a compound according to the invention having hole transporting properties,
- Figure 12 schematically depicts an organic field electric transistor (OFET) in cross-section.
- Figure 13 schematically depicts an inverted OLED in cross-section,
- Figure 14 schematically depicts an OLED in cross-section, and
- Figure 15 schematically depicts a solar cell in cross-section.

The following examples depict some combinations of the inventive core structure with EL substituents. However, exchanges between the exemplary compounds in respect of the EL functional substituents as well as the structure of the core structure in respect of its embodiment as a four- or five-membered ring or a six-membered ring, independently, between each condensed aromatic system and the central atom Z, and embodiments comprising one or both of the condensed aromatic systems as part of higher anellated systems are comprised as embodiments of the invention.

As shown in the following examples, the substituent moieties need not be symmetrical to the central atom. The substituent moieties can comprise different EL functional groups or other residues in various positions of the condensed aromatic systems.

Synthesis of compounds according to the invention can be achieved according to known methods.

### Example 1: Hole transport material

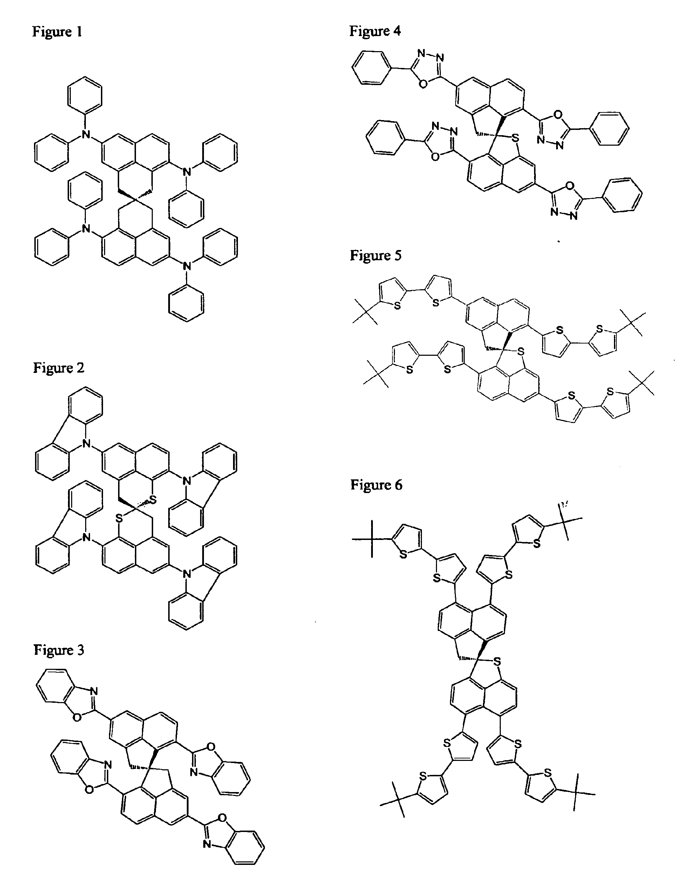

As shown in Figure 1, a transport material based on the core structure according to the invention may be formed by di-substituting each naphthyl group with two diphenylamino groups each. In this embodiment, the core structure according to the invention uses a carbon atom as the central atom Z. Intermediate residues (X, Y and V, W) linking each naphthyl group forming the condensed aromatic system with the central carbon atom are methylene groups, effectively forming a six-membered ring comprising the α and α'carbon atoms, respectively, of each naphthyl group, two intermediate methylene groups and the central carbon atom.

The naphthyl groups are each embodied without further condensed moieties and they are only substituted with EL functional groups in positions 2 and 6, providing the desired charge transporting properties.

### Example 2: Hole transport material

The compound of Figure 2 shows a similar core structure as Figure 1, having two sulfur atoms as intermediate residues to constitute six-membered rings each, formed between the α and α' carbon atoms of the first and second naphthyl group, respectively, including the central carbon atom, and two sulfur atoms.

The naphthyl groups do not form part of a higher anellated aromatic system. The naphthyl groups are each substituted with two carbazole substituents in positions 2 and 6.

### Example 3: Electron transport material

As shown in Figure 3, the core structure of this compound is formed of two opposite naphthyl groups, linked to the central carbon atom forming five-membered rings between the α and α' carbon atoms of the naphthyl groups, respectively, a direct bond to the central carbon atom, the central carbon atom itself and an intermediate methylene group each. Both naphthyl groups are substituted with two moieties each to provide for the desired electronic properties, namely benzoxazole groups.

### Example 4: Electron transport material

The compound depicted in Figure 4 shows the opposite naphthyl groups linked to the central carbon atom, each naphthyl group forming with its α and α' carbon atoms, respectively, a five-membered ring with the central atom. One five-membered ring has a methylene group as the intermediate residue, whereas the opposite five-membered ring has a sulfur atom as the intermediate residue, with the other intermediate residue of each ring being formed by a chemical bond. The substituent groups to the naphthyl groups are 2-phenylbenzoxadiazole groups.

### Example 5: Hole transport material

The compound shown in Figure 5 has an identical core structure to the compound of Example 4, however, the naphthyl groups are substituted with a chain of thienyl groups linked via their α carbon atoms, the terminal thienyl groups are substituted with tertiary butyl groups. This compound is suitable for forming a hole transporting layer in EL devices.

### Example 6: Hole transport material

The compound shown in Figure 6 has a core structure identical to Examples 4 and 5. The two naphthyl groups are substituted on their respective delta carbon atoms. The substituent groups are α, α dithienyl groups with their terminal thienyl group substituted on its α carbon with a tertiary butyl group.

### Example 7: Electron transport material

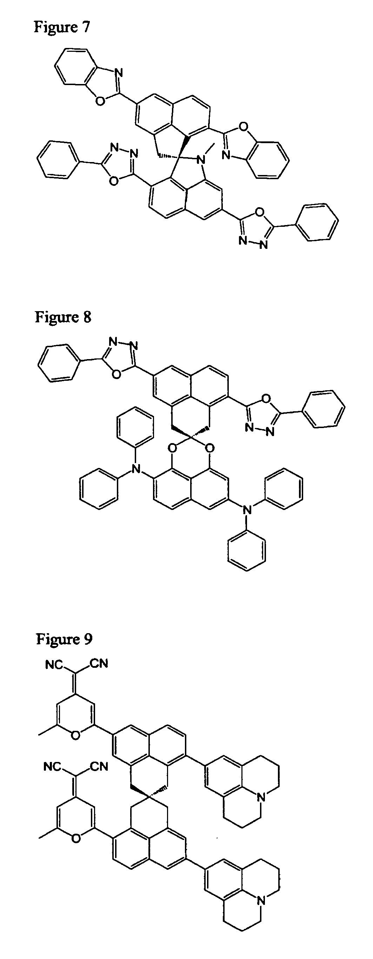

The compound shown in Figure 7 comprises a core structure comprising two opposite naphthyl groups, each forming a five-membered ring with the central carbon atom. One of the five-membered rings contains a methylene group as the intermediate residue, the opposite five-membered ring contains a nitrogen atom, substituted with a naphthyl group as the intermediate residue.

The naphthyl groups are substituted with moieties which confer electron transport properties, namely a 2-phenyloxadiazole substituent on one naphthyl group and a benzoxazole substituent on the opposite naphthyl group. The substituents to the naphthyl moieties are conjugated in positions 3 and 4 of the respective naphthyl groups.

### Example 8: Combined hole and electron transport material

The compound shown in Figure 8 comprises the core structure having six-membered rings of connecting each naphthyl group to the central carbon atom, wherein the six-membered ring comprising the α carbon atoms of one naphthyl group comprises two methylene groups as intermediate residues, the six-membered ring linking the opposite naphthyl group to the same central carbon atom comprises oxygen as such intermediate residues.

The hole transport property is conferred by two diphenylamino substituents, the electron transport property by two 2-phenyloxadiazole substituents.

### Example 9: Emitter compound

The compound shown in Figure 9 has a core structure comprising two naphthyl groups connected to the central carbon atom via six-membered rings, each comprising two methylene groups as intermediate residues.

The naphthyl groups are substituted in position 3 with a dye acceptor moiety, and in position 7 with a dye donor moiety. As a result, a derivative of a merocyanine is formed, in this example corresponding to the known laser dye DCM2.

### Example 10: Hole transporting compound

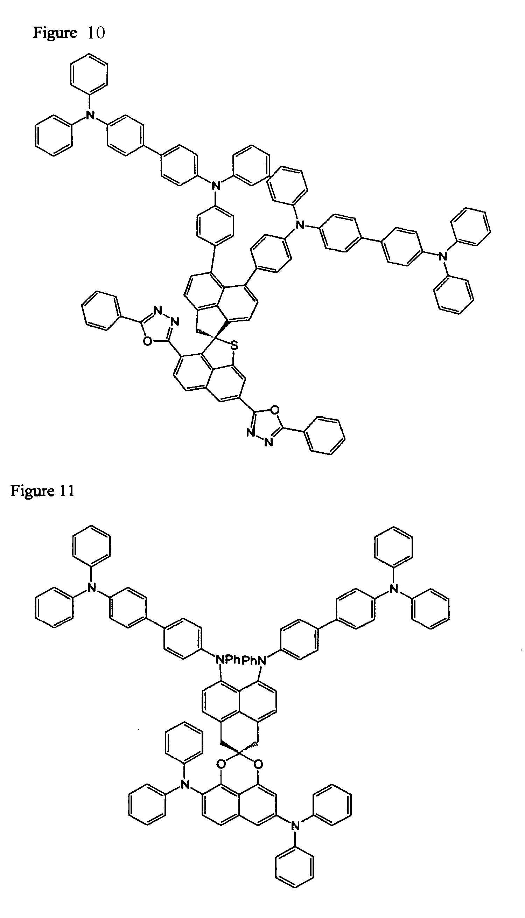

The compound according to Figure 11 comprises a core structure having two opposite naphthyl groups, each connected via a five-membered ring to the central carbon atom, one five-membered ring having a methylene group as the intermediate residue, the opposite five-membered ring having a sulfur atom as the intermediate residue.

One naphthyl group is substituted in positions 1 and 8 with EL functional moieties, namely electron transporting substituent 2-phenylbenzoxadiazole. The opposite naphthyl group is substituted in positions 3 and 7 with diphenylamino substituents, conferring the property of hole transport.

### Example 11: Hole transporting compound

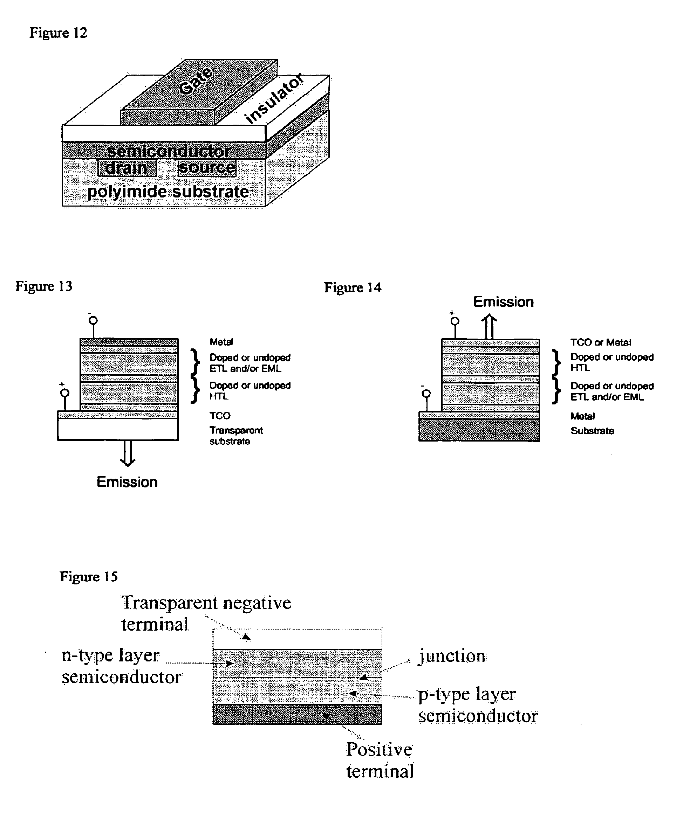

The compound shown in Figure 12 comprises the core structure of two opposite naphthyl groups, each connected via a six-membered ring to the central carbon atom, one six-membered ring comprising methylene groups as the intermediate residues, the opposite six-membered ring comprising oxygen as intermediate residues.

For this compound, hole transporting moieties are present in positions 1 and 8 of one naphthyl group, and diphenylamino groups in positions 2 and 6 of the opposite naphthyl group. However, the positions of the substituent groups on the respective naphthyl groups as well as the substituent groups themselves can be exchanged from one naphthyl group to the other.

### Example 12: EL devices

Compounds comprising the core structure according to the invention can be adapted to have pre-determined electrical and/or optical properties by selecting substituent groups conferring the desired EL properties. Accordingly, compounds according to the invention can be used to form layers in EL devices, wherein the layers require the respective EL properties of the compound. Exemplary EL devices are depicted in Figures 13 to 16, showing an OFET, an inverted OLED in cross-section, an OLED, and a solar cell.

## Claims

1. Compound useful for electronic and/or electrooptic devices, **characterized by** a core structure of formula I : 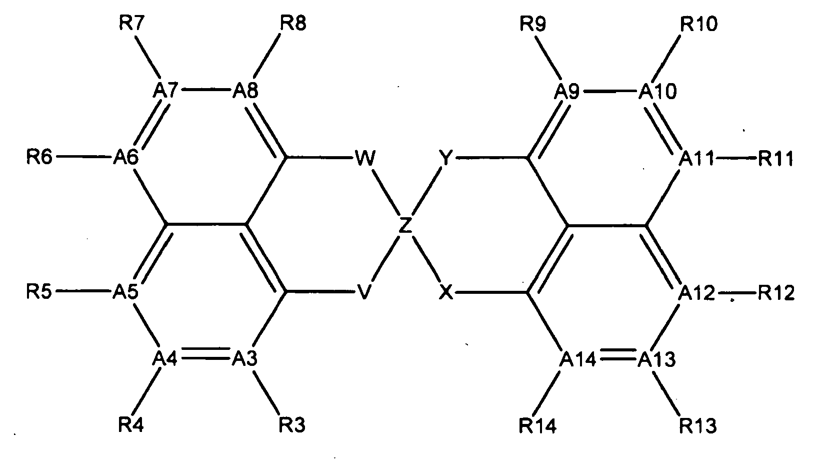
wherein V, W, X and Y are selected from at least divalent atoms or groups, wherein at least one of V, W and X, Y, respectively, is no chemical bond but an at least divalent atom or group,
wherein R3 to R14 are independently selected from (hetero-) alkyls, (hetero-) aryls, -NR'₂, -OR', -SR', -CN, -F, -CF₃, with R' independently selected from substituted or unsubsituted (hetero-)alkyl, (hetero-)aryl or hydrogen, and electrooptically functional groups,
wherein A3 to A 14 are carbon atoms and
wherein Z is a central tetraedric atom.

2. Compound according to claim 1, **characterized in that** V, W, X and Y are selected from the group comprising -S-, -NR-, -O-, a carbonyl group, -SO₂-, and di-substituted silicon, -CRR-, and a chemical bond, with R any (hetero-) alkyl or (hetero-) aryl or hydrogen.

3. Compound according to one of the preceding claims, **characterized in that** two or more of R3 to R14 are condensed arenyl groups or are groups forming a higher condensed analog of the core structure of formula I.

4. Compound according to one of the preceding claims, **characterized in that** central tetraedric atom Z is selected from the group comprising silicon, germanium and carbon.

5. Compound according to one of the preceding claims, **characterized in that** least one of R3 to R14 is selected from hole transporting residues, electron transporting residues and light emitter residues.

6. Compound according to claim 5, **characterized in that** a hole transporting moiety is selected from the group of tris-[(*N,N*-diaryl)amino]-triphenylamines like 4,4',4"-tris[(*N*-(1-naphthyl)-*N*-phenyl-amino-triphenylamine] (1-TNATA) and its derivatives, 4,4',4"-tris[(*N*-(2-naphthyl)-*N*-phenylamino)-triphenylamine] (2-TNATA) or 4,4',4"-tris[(*N*-(3-methylphenyl)-*N*-phenyl-arnino)-triphenylamine] (m-TDATA) and its derivatives, 4,4',4"-tris(carbazole-9-yl)triphenylamines; *N,N,N',N'-tetra* arylbenzidines as *N,N*,*N',N'*-tetraphenylbenzidine and its derivatives, *N,N*'-bis(1-naphthyl)-*N,N*'-diphenyl-benzidine (α-NPD), *N,N*'-di(naphthalene-2-yl)-*N,N*'-diphenyl-benzidine (β-NPD)-, 4,4'-bis(carbazole-9-yl)biphenyl (CBP) and its derivatives, and their heteroatom substituted analogs (e.g. thienyl-, selenyl-, furanyl-derivatives); 4,4'-bis(2,2'-diphenylvinyl)-1,1'-biphenyl (DPVBI); triarylamines and their derivatives, 4,4'-bis(*N,N-*diarylamino)-terphenyls, 4,4'-bis(*N,N*-diarylamino)-quarterphenyls and their homologs and derivatives.

7. Compound according to claim 5 or 6, **characterized in that** an electron transporting moiety is selected from the group of 4,7-diphenyl-1,10-phenanthroline (Bphen) and derivatives thereof as 2,9-dimethyl-4,7-diphenyl-1,10-phenanthroline (BCP), 2,5-diaryloxadiazoles and derivatives thereof as 2-(*p-tert.* -butylphenyl)-5-(*p-*biphenyl)-oxadiazole (PBD), oligo-(benzoxadiazol-2yl)-arenes and derivatives thereof as bis-2,5-(5-*tert.*-butyl-benzoxadiazol-2-yl)-thiophene (BBOT), 1,3-bis[5-(aryl)-1,3,4-oxadiazol-2yl]benzenes and derivatives thereof as 1,3-bis[5-(*p-tert.*-butylphenyl)-1,3,4-oxadiazol-2yl]benzene (OXD-7), 2,5-diaryltriazoles and derivatives like 2-(*p-tert*.-butylphenyl)-5-(*p-*biphenyl)-triazole (TAZ).

8. Compound according to claim 5 to 7, **characterized in that** a light emitter moiety is selected from the group comprising coumarines, rhodamines, merocyanines, cyanines and oxonoles.

9. Compound according to claim 5 to 8, **characterized in that** a light emitter moiety is formed by two or more of R3 to R14 that are condensed arenyl groups.

10. Compound according to one of the preceding claims useful for electronic and/or electrooptic devices, represented by one of the following formulae:

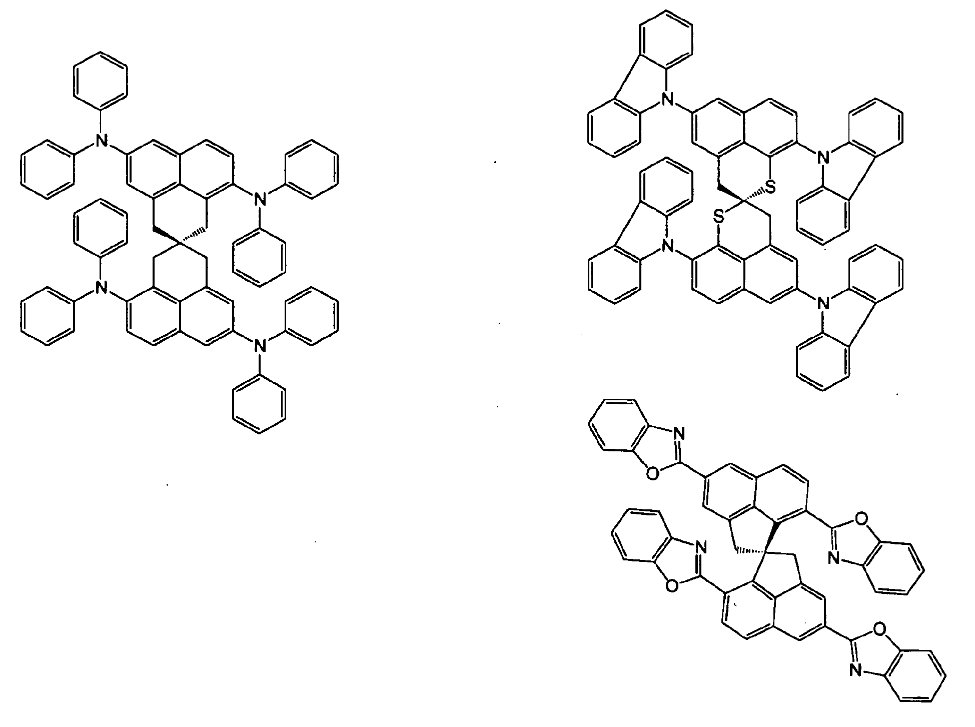

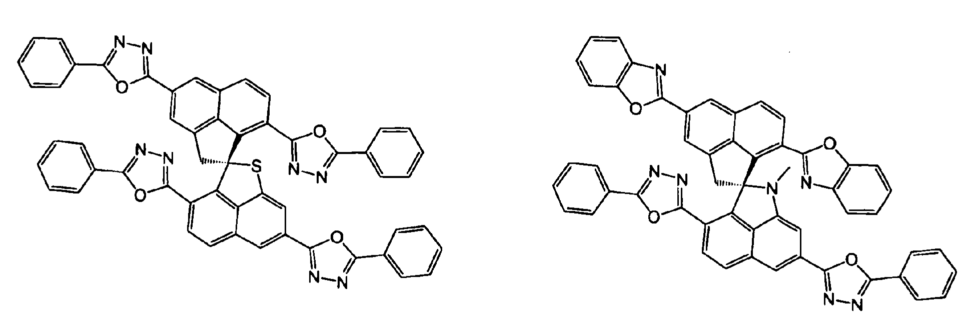

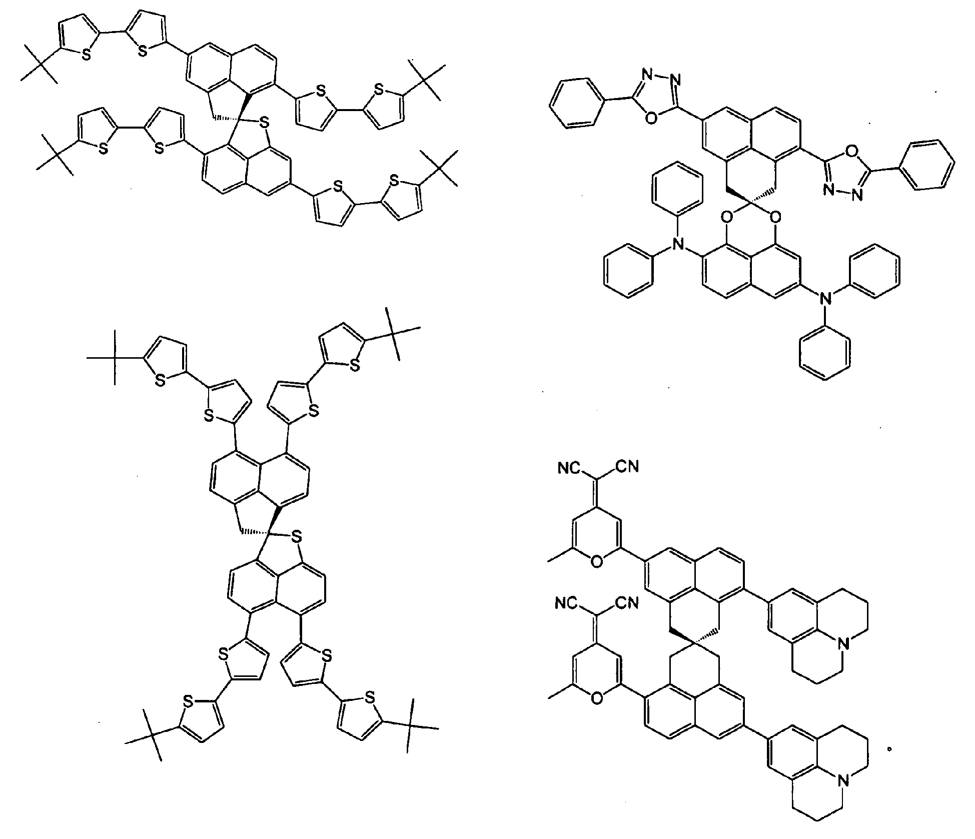

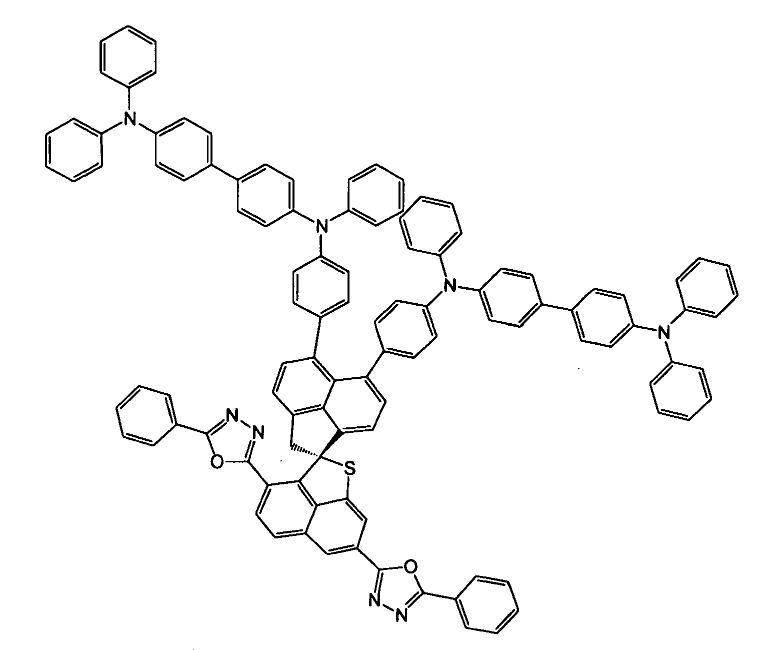

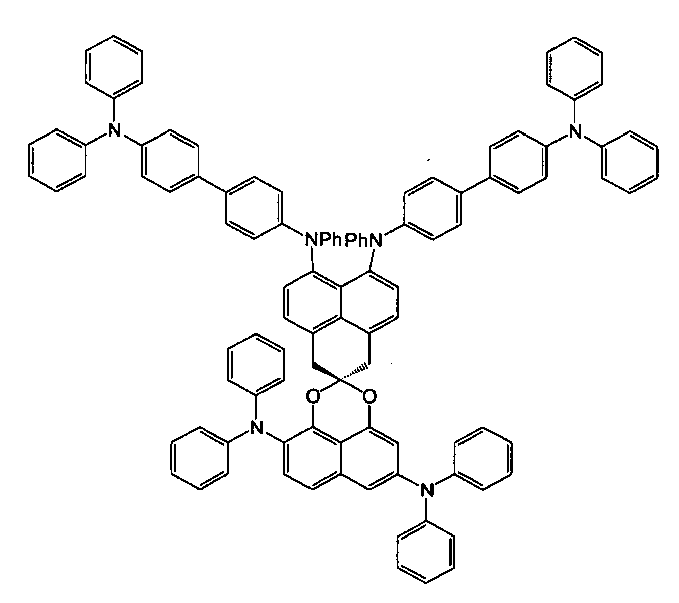

11. Compound according to one of the preceding claims, **characterized in that** the compound is a hole transporter, electron transporter and/or light emitter.

12. Process for synthesis of compounds useful for electroluminescent devices, **characterized by** generating a compound according to one of the preceding claims.

13. Electronic, electrooptic or electroluminescent device, **characterized by** comprising a compound according to claims 1 to 11.

## Patentansprüche

1. Verbindung, die für elektronische und/oder elektrooptische Geräte verwendbar ist, **gekennzeichnet durch** eine Kernstruktur gemäß Formel I: 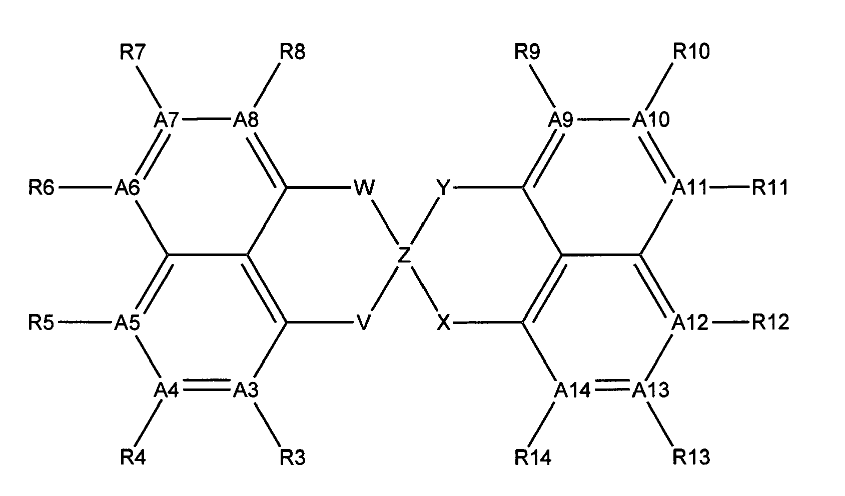
bei der V, W, X und Y aus wenigstens divalenten Atomen oder Gruppen ausgewählt sind,
bei der wenigstens eines von V, W bzw. X, Y keine chemische Bindung ist, sondern eine wenigstens divalentes Atom oder eine Gruppe,
bei der R3 bis R14 unabhängig ausgewählt sind aus (Hetero-)Alkylen, (Hetero-) Arylen, -NR'₂, -OR', -SR', -CN, -F, -CF₃, wobei R' unabhängig unter substituiertem oder unsubstituiertem (Hetero-)Alkyl, (Hetero-)Aryl oder Wasserstoff und elektrooptisch funktionalen Gruppen gewählt ist,
bei der A3 bis A14 Kohlenstoffatome sind und
bei der Z ein zentrales tetraetisches Atom ist.

2. Verbindung nach Anspruch 1, **dadurch gekennzeichnet, dass** V, W, X und Y aus der Gruppe ausgewählt sind, die -S-, -NR-, -O-, einen Carbonylgruppe, -SO₂-, und disubstituiertes Silicium, -CRR- und eine chemische Bindung umfasst, wobei R jedes (Hetero-)Alkyl oder (hetero-)Aryl oder Wasserstoff ist.

3. Verbindung nach einem der voranstehenden Ansprüche, **dadurch gekennzeichnet, dass** zwei oder mehr von R3 bis R14 kondensierte Arenylgruppen sind oder Gruppen, die ein höher kondensiertes Analogon der Kernstruktur von Formel I bilden.

4. Verbindung nach einem der voranstehenden Ansprüche, **dadurch gekennzeichnet, dass** das zentrale tetraetische Atom Z aus der Gruppe ausgewählt ist, die Silicium, Germanium und Kohlenstoff umfasst.

5. Verbindung nach einem der voranstehenden Ansprüche, **dadurch gekennzeichnet, dass** wenigstens eines von R3 bis R14 aus Lochtransportgruppen, Elektronentransportgruppen und Lichtemittergruppen ausgewählt ist.

6. Verbindung nach Anspruch 5, **dadurch gekennzeichnet, dass** eine Lochtransportgruppe aus der Gruppe von Tris-[(*N,N*-diaryl)amino]-triphenylaminen, wie 4,4',4"-Tris[(*N-*(1-naphthyl)-*N*-phenylamino-triphenylamin] (1-TNATA) und dessen Derivaten, 4,4',4"-Tris[(*N*-(2-naphthyl)-*N*-phenylamino)-triphenylamin] (2-TNATA) oder 4,4',4"-Tris[(*N*-(3-methylphenyl)-*N*-phenylamino)-triphenylamin] (m-TDATA) und dessen Derivaten, 4,4',4"-Tris(carbazol-9-yl)triphenylaminen; *N,N,N',N'-*Tetraarylbenzidinen, wie *N,N*,*N',N'*-Tetraphenylbenzidin und dessen Derivaten, *N,N*'-Bis(1-naphthyl)-*N,N*'-diphenylbenzidin (α-NPD), *N,N*'-Di(naphthalen-2-yl)-*N,N*'-diphenylbenzidin (β-NPD), 4,4'-Bis(carbazol-9-yl)biphenyl (CBP) und dessen Derivaten, und deren heteroatomsubstituierten Analoga (z.B. Thienyl-, Selenyl-, Furanyl-derivativen); 4,4'-Bis(2,2'-diphenylvinyl)-1,1'-biphenyl (DPVBI); Triarylaminen und deren Derivaten, 4,4'-Bis(*N,N*-diarylamin)-terphenylen, 4,4'-Bis(*N,N*-Diarylamin)-quaterphenylen und deren Homologen und Derivaten ausgewählt ist.

7. Verbindung nach Anspruch 5 oder 6, **dadurch gekennzeichnet, dass** eine Elektronentransportgruppe aus der Gruppe von 4,7-Diphenyl-1,10-phenanthrolin (Bphen) und dessen Derivaten, wie 2,9-Dimethyl-4,7-diphenyl-1,10-phenanthrolin (BCP), 2,5-Diaryloxadiazolen und dessen Derivaten, wie 2-(*p-tert.*-Butylphenyl)-5-(*p-*biphenyl)-oxadiazol (PBD), oligo-(Benzoxadiazol-2yl)-arenen und deren Derivaten, wie Bis-2,5-(5-*tert.*-Butylbenzoxadiazol-2-yl)-thiophen (BBOT), 1,3-bis[5-(Aryl)-1,3,4-oxadiazol-2y1]benzenen und deren Devivaten, wie 1,3-bis[5-(*p*-*tert.*-Butylphenyl)-l,3,4-oxadiazol-2yl]benzen (OXD-7), 2,5-Diaryltriazolen und Derivaten, wie 2-(*p-tert.*-Butylphenyl)-5-(*p-*biphenyl)-triazol (TAZ) ausgewählt ist.

8. Verwendung nach Anspruch 5 bis 7, **dadurch gekennzeichnet, dass** eine Lichtemittergruppe aus der Gruppe ausgewählt ist, die Cumarine, Rhodamine, Merocyanine, Cyanine und Oxonole umfasst.

9. Verbindung nach Anspruch 5 bis 8, **dadurch gekennzeichnet, dass** eine Lichtemittergruppe von zwei oder mehr von R3 bis R14 gebildet ist, die kondensierte Arenylgruppen sind.

10. Verbindung nach einem der voranstehenden Ansprüche, die für elektronische und/oder elektrooptische Geräte geeignet sind, dargestellt durch eine der folgenden Formeln:

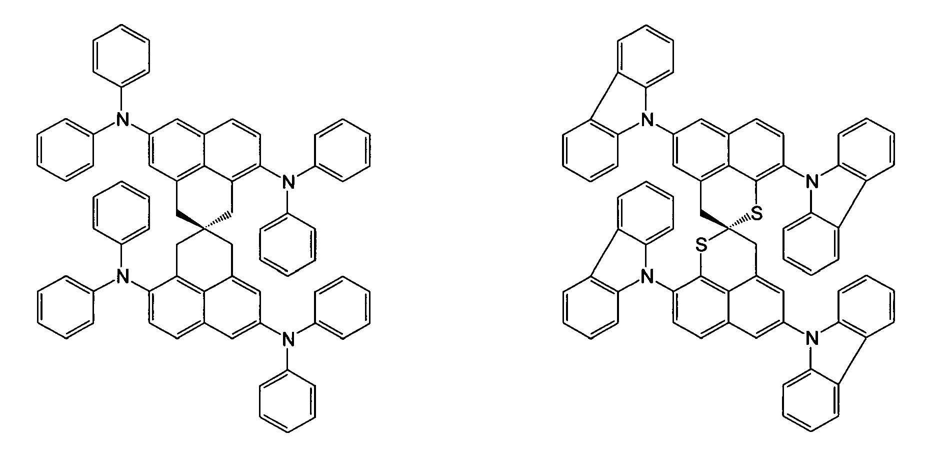

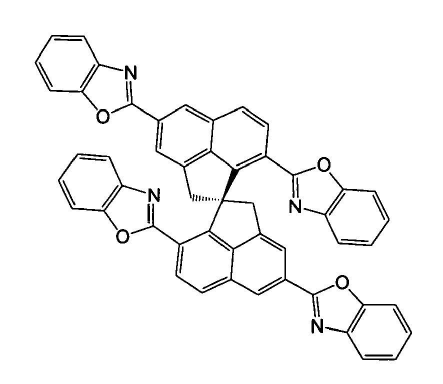

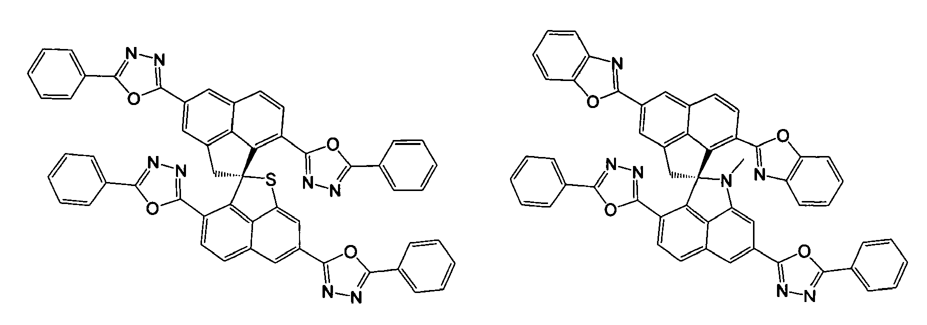

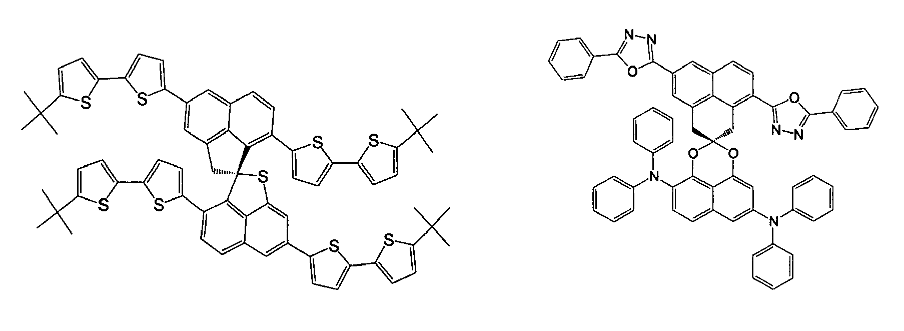

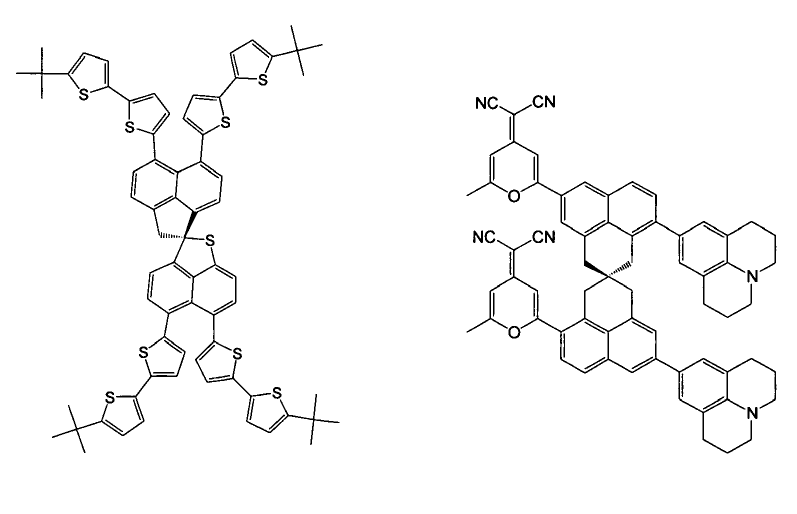

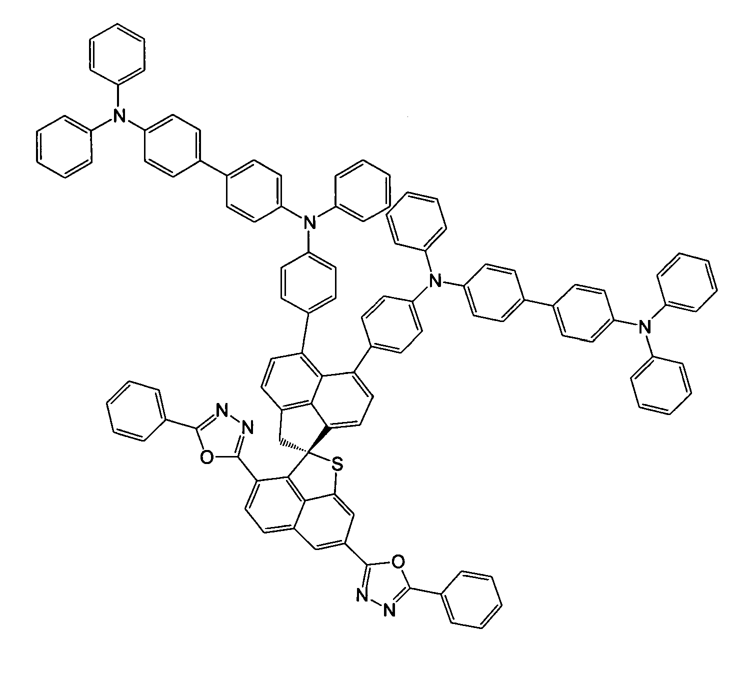

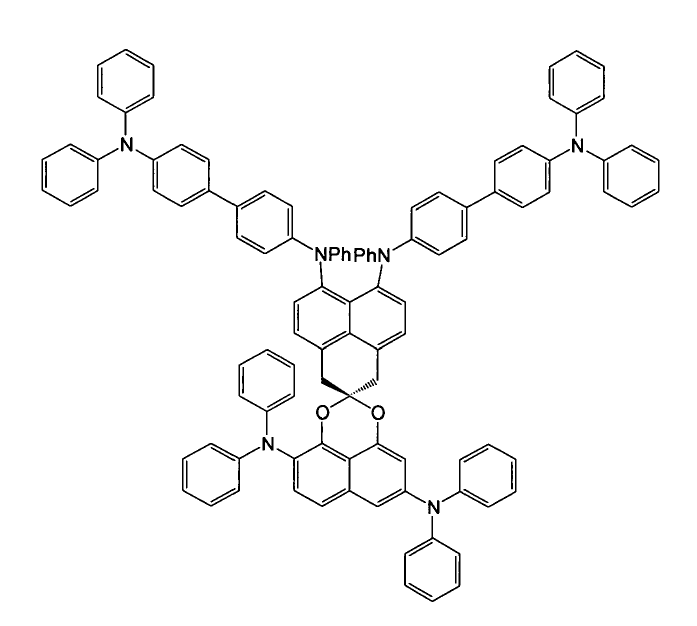

11. Verbindung nach einem der voranstehenden Ansprüche, **dadurch gekennzeichnet, dass** die Verbindung ein Lochtransporter, ein Elektronentransporter und/oder Lichtemitter ist.

12. Verfahren zur Synthese von Verbindungen, die für elektrolumineszente Geräte geeignet sind, **gekennzeichnet durch** das Erzeugen einer Verbindung nach einem der voranstehenden Ansprüche.

13. Elektronisches, elektrooptisches oder elektrolumineszentes Gerät, **gekennzeichnet durch** das Umfassen einer Verbindung gemäß einem der Ansprüche 1 bis 11.

## Revendications

1. Composé utilisé pour des dispositifs électroniques et/ou électro-optiques, **caractérisé par** une structure de noyau de formule I : 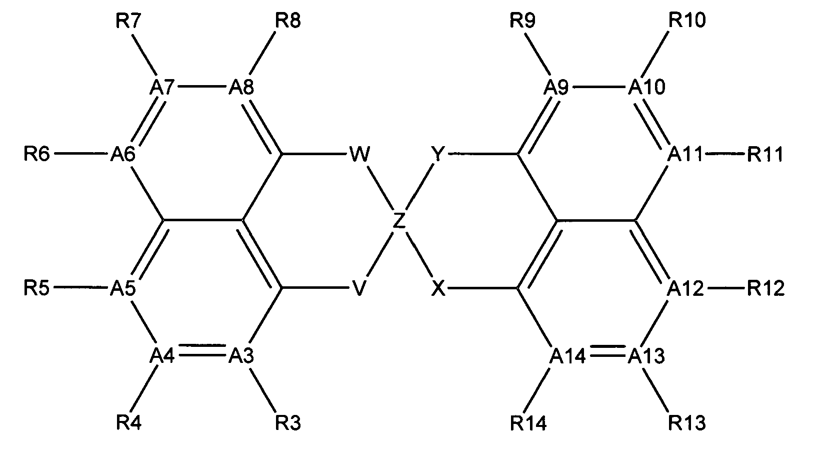
dans laquelle V, W, X et Y sont sélectionnés parmi des atomes ou des groupes au moins divalents,
dans laquelle au moins l'un de V, W et X, Y, respectivement, n'est pas une liaison chimique, mais un atome ou un groupe au moins divalent,
dans laquelle R3 à R14 sont sélectionnés indépendamment parmi les (hétéro-) alkyles, les (hétéro-) aryles, -NR'₂, -OR', -SR', -CN, -F, -CF₃, R' étant sélectionné indépendamment parmi l'(hétéro-)alkyle, l'(hétéro-)aryle or l'hydrogène substitué ou non-substitué , et les groupes fonctionnels sur le plan électro-optique,
dans laquelle A3 à A14 sont des atomes de carbone', et
dans laquelle Z est un atome tétraédrique central.

2. Composé selon la revendication 1, **caractérisé en ce que** V, W, X et Y sont sélectionnés dans le groupe comprenant -S-, -NR-, -O-, un groupe carbonyle, -SO₂-, et un silicium di-substitué, -CRR-, et une liaison chimique, R étant l'un quelconque de l'(hétéro-) alkyle ou l'(hétéro-) aryle ou l'hydrogène.

3. Composé selon l'une des revendications précédentes, **caractérisé en ce que** 'deux' ou plus, parmi R3 à R14 sont des groupes arényle condensés ou sont des groupes formant un analogue plus fortement condensé de la structure de noyau de formule I.

4. Composé selon l'une des revendications précédentes, **caractérisé en ce que** l'atome tétraédrique central Z est sélectionné dans le groupe comprenant le silicium, le germanium et le carbone.

5. Composé selon l'une des revendications précédentes, **caractérisé en ce qu'**au moins l'un de R3 à R14 est sélectionné parmi les résidus de transport de trou, les résidus de transport d'électrons et les résidus d'émetteurs de lumière.

6. Composé selon la revendication 5, **caractérisé en qu'**un groupe fonctionnel de transport de trou est sélectionné dans le groupe des tris-[(*N,N*-diaryl)amino]-triphénylamines telles que 4,4',4"-tris [(*N*-(1-naphthyl)-*N*-phényl-amino-triphénylamine] (1-TNATA) et ses dérivés, 4,4',4"-tris[(*N*-(2-naphthyl)-*N*-phényl amino)-triphénylamine] (2-TNATA) ou 4,4',4"-tris[(*N*-(3-méthylphényl)-*N*-phényl-amino)-triphénylamine] (m-TDATA) et ses dérivés, 4,4',4"-tris(carbazole-9-yl)triphénylamines; *N,N*,*N',N'*-tetra arylbenzidines sous la forme de *N,N,N',N'-*tetraphénylbenzidine et ses dérivés, *N,N*'-bis(1-naphthyl)-*N,N*'-diphényl-benzidine (α-NPD), *N,N*'-di(naphthalène-2-yl)-*N,N*'-diphényl-benzidine (β-NPD), 4,4'-bis(carbazole-9-yl)biphényl (CBP) et ses dérivés, et leurs analogues substitués à substitution hétéroatome (par exemple les dérivés thiényle, sélényle, furanyle); 4,4'-bis(2,2'-diphénylvinyl)-1,1'-biphényle (DPVBI); triarylamines et leurs dérivés, 4,4'-bis(*N,N*-diarylamino)-terphényles, 4,4'*-*bis*(N*,*N*-diarylamino)-quarterphényles et leurs homologues et dérivés.

7. Composé selon les revendications 5 ou 6, **caractérisé en ce qu'**un groupe fonctionnel de transport d'électron est sélectionné dans le groupe des 4,7-diphényl-1,10-phenanthroline (Bphen) et ses dérivés sous la forme de 2,9-diméthyl-4,7-diphényl-1,10-phenanthroline (BCP), 2,5-diaryloxadiazoles et leurs dérivés sous la forme de 2-(*p-tert.*-butylphényl)-5-(*p-*biphényl)-oxadiazole (PBD), oligo-(benzoxadiazol-2yl)-arènes et leurs dérivés sous la forme de bis-2,5-(5-*tert.*-butyl-benzoxadiazol-2-yl)-thiophène (BBOT), 1,3-bis[5-(aryl)-1,3,4-oxadiazol-2yl]benzènes et leurs dérivés sous la forme de 1,3-bis[5-(*p-tert.*-butylphényl)-1,3,4-oxadiazol-2yl]benzène (OXD-7), 2,5-diaryltriazoles et leurs dérivés tels que 2-(*p-tert.*-butylphényl)-5-(*p-*biphényl)-triazole (TAZ).

8. Composé selon les revendications 5 à 7, **caractérisé en ce qu'**un groupe fonctionnel d'émetteur de lumière est sélectionné dans le groupe comprenant les coumarines, les rhodamines,les merocyanines, les cyanines et les oxonoles.

9. Composé selon les revendications 5 à 8, **caractérisé en ce qu'**un groupe fonctionnel d'émetteur de lumière est formé de deux, ou plus, parmi R3 à R14 qui sont des groupes arenyle condensés.

10. Composé selon l'une des revendications précédentes utilisé pour des dispositifs électroniques et/ou électro-optiques, représentés par l'une des formules suivantes :

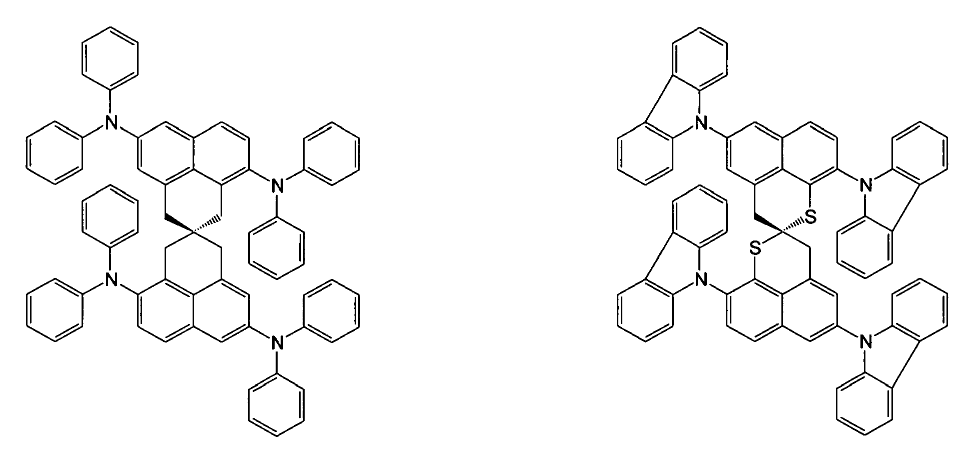

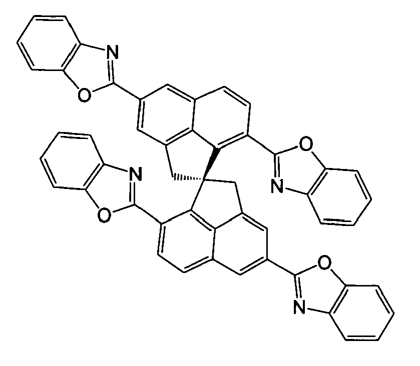

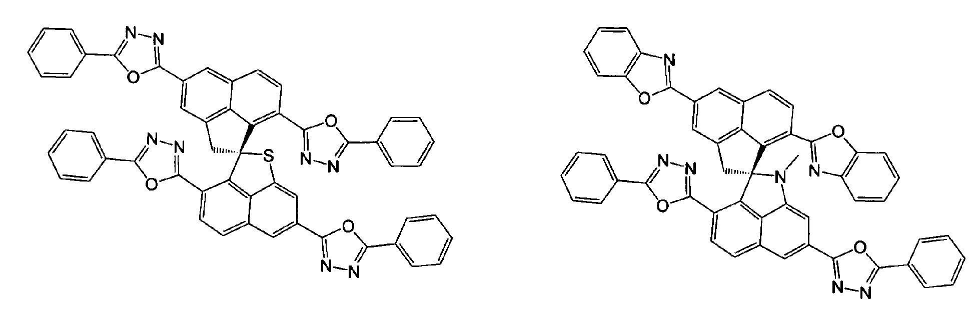

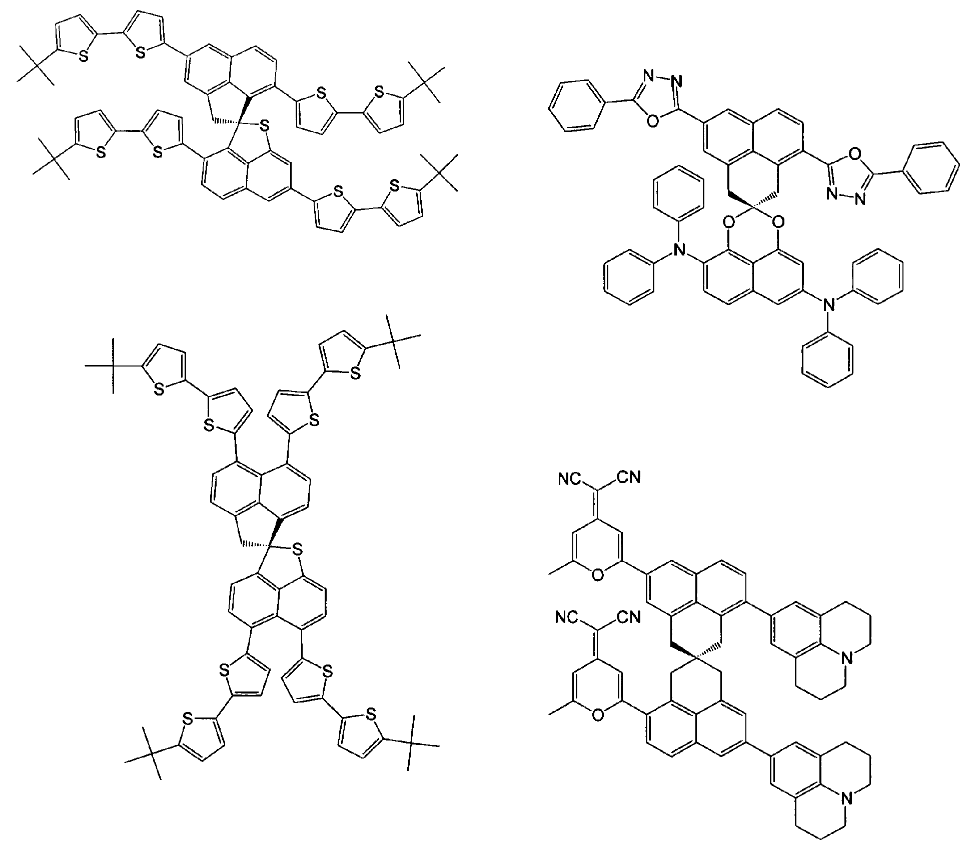

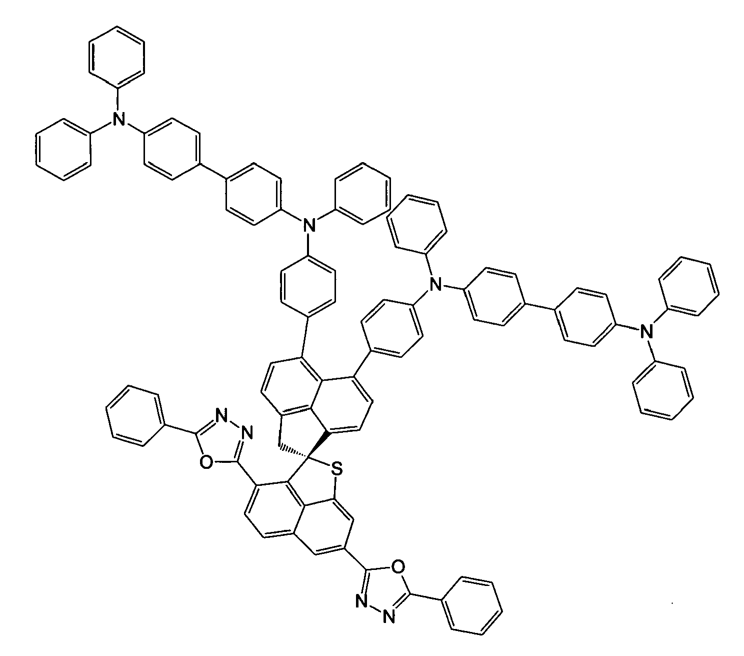

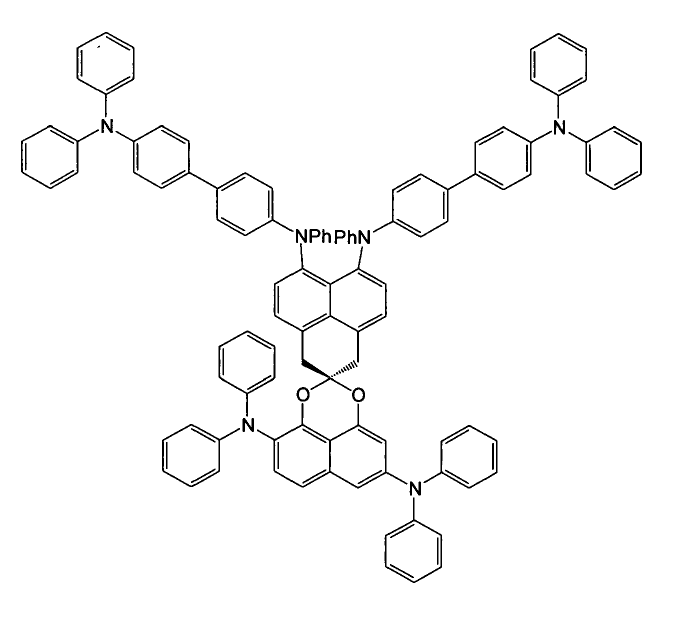

11. Composé selon l'une des revendications précédentes, **caractérisé en ce que** le composé est un transporteur de trou, un transporteur d'électrons et/ou un émetteur de lumière.

12. Procédé de synthèse de composés utilisés dans des dispositifs électroluminescents, **caractérisé par** la génération d'un composé selon l'une des revendications précédentes.

13. Dispositif électronique, électro-optique ou électroluminescent, **caractérisé en ce qu'**il comprend un composé selon les revendications 1 à 11.
